# EUROPEAN PATENT APPLICATION

(11) **EP 2 138 139 A2**
(43) Date of publication of application: **30.12.2009**
(21) Application number: 09162540.0
(22) Date of filing: 12.06.2009
(51) Int. Cl.: A61F 13/02

(54) **Device for treating a wound with means for generating negative pressure**

(30) Priority: 27.06.2008 IT MI20081177
(71) Applicant: Eurosets S.r.l., 41036 Medolla (MO) (IT)
(72) Inventor: Petralia, Antonio, 48022, Lugo RA (IT); Fontanili, Paolo, 42015, Correggio RE (IT)
(74) Representative: Modiano, Micaela Nadia

(57) **Abstract**

A device for treating a wound, comprising means (1) adapted to create a negative pressure at a region that comprises the wound (2) and is protected by sealing means (3) and further comprising shielding means (5) that are adapted to be arranged in contact with the wound (2), the shielding means (5) being provided by means of a deformable layer (6) of treated polymeric nonwoven fabric.

## Description

The present invention relates to a device for treating a wound with means for generating negative pressure.

Devices are already known which are used to accelerate the healing of wounds, which comprise means adapted to create a negative pressure at a region that comprises the wound and is protected by way of sealing means that are fixed to the skin all around the wound.

Such devices further provide for the presence of shielding means, which are arranged in contact with the wound and which in the background art assume the most different shapes, all of which, however, are not entirely satisfactory.

The aim of the present invention is to devise a device that is provided with shielding means that ensure a drastic reduction in the release of filaments that remain attached to the wound upon separation of the dressing.

Within this aim, an object of the invention is to provide a device in which the shielding means have a high degree of biocompatibility.

This aim and these and other objects are achieved by a device for treating a wound, according to the invention, comprising means adapted to create a negative pressure at a region that comprises the wound and is protected by sealing means and further comprising shielding means that are adapted to be arranged in contact with said wound, **characterized in that** said shielding means are provided by way of a deformable layer of treated polymeric nonwoven fabric.

Further characteristics and advantages of the present invention will become better apparent from the description of two preferred but not exclusive embodiments thereof, illustrated by way of non-limiting example in the accompanying drawings, wherein:
Figure 1 is a view of a device for operating a wound;
Figure 2 is a perspective view of a first embodiment of the wound shielding means;
Figure 3 is a sectional view taken along the line III-III of Figure 2;
Figure 4 is a sectional view of another embodiment of the shielding means.

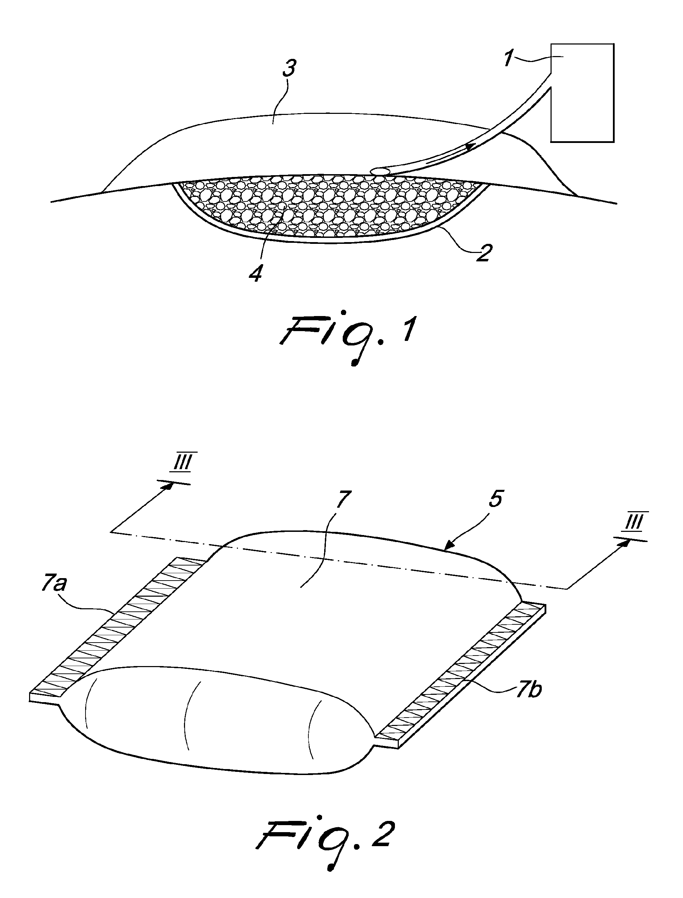

Figure 1 illustrates a conventional device, which comprises means 1 adapted to create a negative pressure at a region that comprises the wound 2 and is protected by sealing means 3 that are fixed to the skin around the wound; shielding means 4 are also provided which are arranged in contact with the wound.

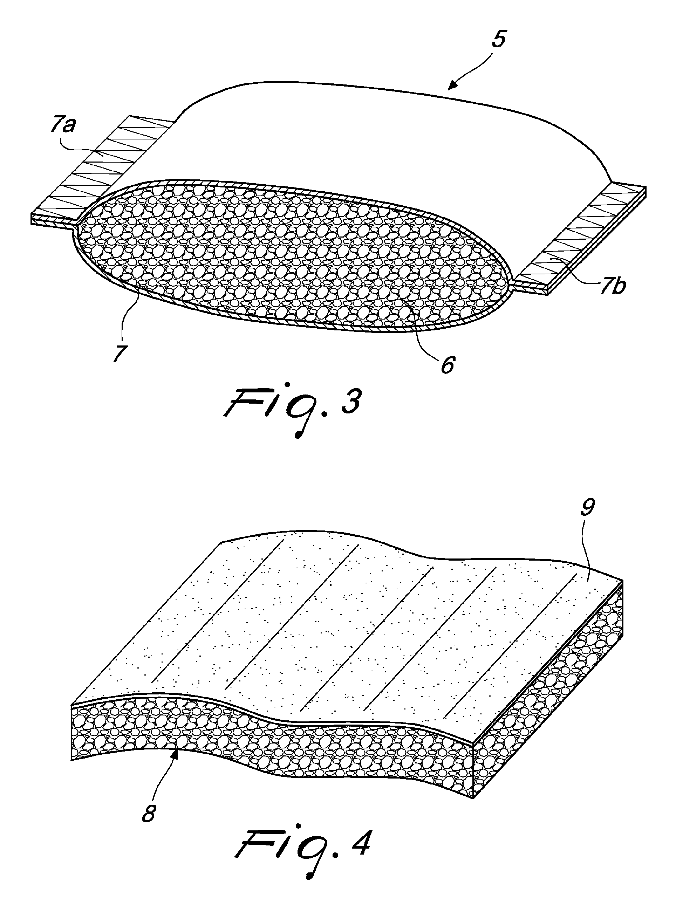

With reference to Figures 2 and 3, shielding means, generally designated by the reference numeral 5, comprise a layer 6 made of nonwoven fabric, NWF, for example of the polymeric type, which is treated by covering with fabric in the form of a tube 7 that is closed at two sides by means of stitched seams 7a, 7b.

Advantageously, the polymeric NWF that is used is of the thermobonded type.

The treatment of the polymeric NWF layer by covering with fabric can have several variations with respect to the embodiment described now.

It is in fact possible to adopt two sheets of fabric that are joined by sewing at the perimeter or a pouch of fabric that is closed by sewing on one side, and it is further possible to provide for the presence, in an intermediate position, of at least one pair of stitched seams that are side by side and spaced in order to allow cutting between them, so as to provide shielding means of optimized dimensions with respect to the dimensions of the wound to be treated.

It is also possible to use a sheet of fabric that is jointly connected by means of adhesive or without added material at at least one face of the layer of polymeric NWF.

Advantageously, in all the cases described above the fabric used is of the polymeric type.

It clearly appears that covering the layer of polymeric NWF by means of fabric makes it highly unlikely to release NWF filaments onto the wound upon removal of the shielding.

Figure 4 illustrates a variation of the device according to the invention, in which there is, at one face of the layer 8 made of polymeric NWF, a surface layer 9 provided by means of a substance that is different and jointly connected to such face.

There are many possible choices for the substance for providing the layer 9, and it is possible to adopt for example a substance that is adapted to biomimic NWF so as to make it similar to membranes of animal cells, thus obtaining, in addition to the substantial blocking of NWF filaments, also high biocompatibility features.

Further possibilities of choice can be directed toward substances of the oily type, such as can be for example silicone, or toward substances in a water-oil emulsion, or also toward substances constituted by a polymer, such as polyurethane, dissolved in a solvent.

A further variation of the treatment of the polymeric NWF can consist, according to the invention, in immersing it in a liquid substance affecting all of its mass, obtaining once again an absolutely drastic reduction in the release of NWF filaments on the wound.

The selected substance can be of the type adapted to biomimic NWF so as to make it similar to membranes of animal cells, but the selection can also be oriented toward substances of the oily type, such as for example silicone, or in a water-oil emulsion.

The described invention is susceptible of numerous other modifications and variations, all of which are within the scope of the appended claims; all the details may further be replaced with other technically equivalent elements.

The disclosures in Italian Patent Application no. MI2008A001177, from which this application claims priority, are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. The device for treating a wound, comprising means adapted to create a negative pressure at a region that comprises the wound and is protected by sealing means and further comprising shielding means that are adapted to be arranged in contact with said wound, **characterized in that** said shielding means are provided by way of a deformable layer of treated polymeric nonwoven fabric.

2. The device according to claim 1, **characterized in that** the shielding means are provided by means of a deformable layer of treated thermobonded polymeric nonwoven fabric.

3. The device according to one or more of the preceding claims, **characterized in that** the layer of nonwoven fabric is covered at at least one face by means of fabric.

4. The device according to one or more of the preceding claims, **characterized in that** the layer of nonwoven fabric is covered at at least one face by means of polymeric fabric.

5. The device according to one or more of the preceding claims, **characterized in that** the fabric that covers the layer of nonwoven fabric comprises two sheets joined by sewing at the perimeter.

6. The device according to one or more of the preceding claims, **characterized in that** the fabric for covering the layer of nonwoven fabric comprises a tubular element that is closed by sewing on two sides.

7. The device according to one or more of the preceding claims, **characterized in that** the fabric for covering the layer of nonwoven fabric comprises a pouch that is closed by sewing on one side.

8. The device according to one or more of the preceding claims, **characterized by** the presence, in an intermediate position, of at least one pair of stitched seams that are arranged side-by-side and spaced in order to allow cutting between them.

9. The device according to one or more of the preceding claims,
**characterized by** the presence of a sheet of fabric that is jointly connected to the layer of nonwoven fabric at at least one face.

10. The device according to one or more of the preceding claims, **characterized by** the presence of a sheet of fabric that is jointly connected by adhesive bonding to the layer of nonwoven fabric at at least one face.

11. The device according to one or more of the preceding claims, **characterized by** the presence of a sheet of fabric that is jointly connected without added material to the layer of nonwoven fabric at at least one face.

12. The device according to claims 1 and 2, **characterized in that** the layer of nonwoven fabric is treated by immersion in a liquid substance.

13. The device according to claims 1 and 2, **characterized in that** the layer of nonwoven fabric is treated by immersion in a liquid substance that is adapted to biomimic nonwoven fabric so as to make it similar to membranes of animal cells.

14. The device according to claims 1 and 2, **characterized in that** the layer of nonwoven fabric is treated by immersion in a liquid substance of the oily type.

15. The device according to claims 1 and 2, **characterized in that** the layer of nonwoven fabric is treated by immersion in a liquid substance constituted by silicone.

16. The device according to claims 1 and 2, **characterized in that** the layer of nonwoven fabric is treated by immersion in a liquid substance in a water-oil emulsion.

17. The device according to claims 1 and 2, **characterized by** the presence, at at least one face of the layer of nonwoven fabric, of a surface layer of a different substance that is jointly connected to said face.

18. The device according to claims 1 and 2, **characterized by** the presence, at at least one face of the layer of nonwoven fabric, of a surface layer of a different substance that is jointly connected to said face and is adapted to biomimic the nonwoven fabric so as to make it similar to membranes of animal cells.

19. The device according to claims 1 and 2, **characterized by** the presence, at at least one face of the layer of nonwoven fabric, of a surface layer of a different substance that is jointly connected to said face of the oily type.

20. The device according to claims 1 and 2, **characterized by** the presence, at at least one face of the layer of nonwoven fabric, of a surface layer of a different substance that is jointly connected to said face constituted by silicone.

21. The device according to claims 1 and 2, **characterized by** the presence, at at least one face of the layer of nonwoven fabric, of a surface layer of a different substance that is jointly connected to said face in a water-oil emulsion.

22. The device according to claims 1 and 2, **characterized by** the presence, at at least one face of the layer of nonwoven fabric, of a surface layer of a different substance that is jointly connected to said face and is constituted by a polymer dissolved in solvent.
